(19) Europäisches Patentamt
European Patent Office
Office européen des brevets

(11) Veröffentlichungsnummer: **0 068 138**
**A1**

(12) # EUROPÄISCHE PATENTANMELDUNG

(21) Anmeldenummer: **82104460.9**

(22) Anmeldetag: **21.05.82**

(51) Int. Cl.³: **C 07 D 249/08**, A 01 N 43/64

(30) Priorität: **23.05.81 DE 3120665**

(43) Veröffentlichungstag der Anmeldung: **05.01.83**
**Patentblatt 83/1**

(84) Benannte Vertragsstaaten: **BE CH DE FR GB IT LI NL**

(71) Anmelder: **HOECHST AKTIENGESELLSCHAFT,**
**Postfach 80 03 20, D-6230 Frankfurt/Main 80 (DE)**

(72) Erfinder: **Koch, Manfred, Dr., Kreuzheck 2,**
**D-6239 Eppstein/Taunus (DE)**
Erfinder: **Kübel, Börries, Dr., Kuckucksweg 14,**
**D-6233 Kelkheim (Taunus) (DE)**
Erfinder: **Bauer, Klaus, Dr., Kolpingstrasse 7,**
**D-6054 Rodgau (DE)**
Erfinder: **Bieringer, Hermann, Dr., Eichenweg 26,**
**D-6239 Eppstein/Taunus (DE)**

(54) **Neue Chloracetanilide, Verfahren zu ihrer Herstellung und sie enthaltende herbizide Mittel.**

(57) Verbindungen der Formel I ($R_1$ und $R_3$ : Alkyl, $R_2$ und $R_4$ : H oder Alkyl und $R_5$ : H oder $CH_3$) sind wirksame Herbizide und Wachstumsregulatoren.

EP 0 068 138 A1

Neue Chloracetanilide, Verfahren zu ihrer Herstellung und sie enthaltende herbizide Mittel

Gegenstand der vorliegenden Anmeldung sind neue Chloracetanilide der allgemeinen Formel I

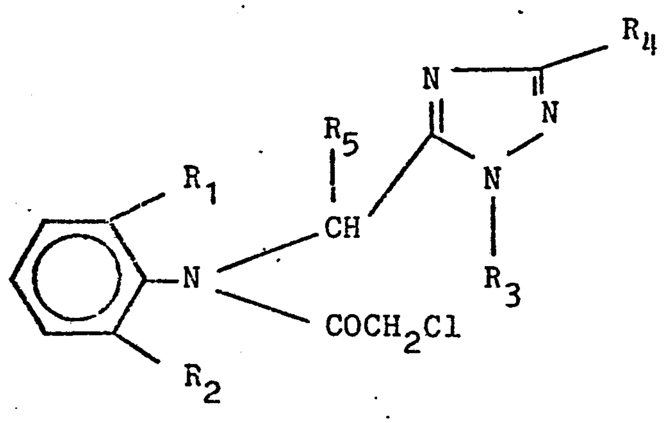

I

worin

$R_1$ Methyl, Äthyl oder Isopropyl,
$R_2$ Wasserstoff, Methyl, Äthyl oder Isopropyl,
$R_3$ $(C_1-C_3)$-Alkyl,
$R_4$ Wasserstoff oder $(C_1-C_3)$-Alkyl, und
$R_5$ Wasserstoff oder Methyl bedeuten.

Die Chloracetanilide der Formel I werden hergestellt, indem man N-substituierte Aniline der Formel II

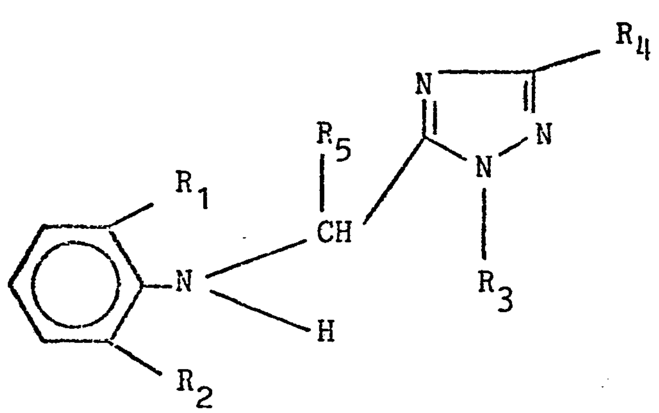

II

mit Chloracetylierungsmitteln behandelt.

BAD ORIGINAL

Verbindungen der Formeln I und II werden in der DE-OS 28 42 284 nebst anderen heterocyclisch substituierten Halogenacetaniliden genannt. In der älteren Anmeldung fehlen jedoch Angaben darüber, wie Verbindungen der Formel I und II erhalten werden können. Tatsächlich waren derartige Verbindungen bisher nicht herstellbar, da die als Ausgangsstoffe für ihre Herstellung erforderlichen 5-Halogenalkyl-1,2,4-triazole nicht verfügbar waren. Solche Ausgangsstoffe sind erst durch das Verfahren der Anmeldung P 31 18 258.5 (Umsetzung von Amidrazonen der Formel $H_2N-CR_4=N-NHR_3$ mit Halogencarbonsäurehalogeniden der Formel $R_5-CH(Hal)-CO-Hal$ und nachfolgenden Ringschluß) zugänglich geworden.

Die Umsetzung II ⟶ I kann in An- oder Abwesenheit von gegenüber den Reaktionsteilnehmern inerten Lösungs- oder Verdünnungsmitteln durchgeführt werden. Es kommen bspw. in Frage: aliphatische, aromatische oder halogenierte Kohlenwasserstoffe wie Benzol, Toluol, Xylole, Petroläther, Chlorbenzol, Methylenchlorid, Äthylenchlorid, Chloroform; Äther wie Dialkyläther, dioxan, Tetrahydrofuran; Nitrile wie Acetonitril, N,N-dialkylierte Amide wie Dimethylformamid; ferner Dimethylsulfoxid sowie Gemische dieser Lösungsmittel untereinander. Die Umsetzungstemperaturen liegen zwischen 0°C und dem Siedepunkt des verwendeten Lösungsmittels, vorzugsweise zwischen 20 und 140°C.

Geeignete Chloracetylierungsmittel sind Chloressigsäurehalogenide wie Chloressigsäurechlorid, Chloressigsäurebromid, Chloressigsäureanhydrid, Chloressigsäure und Chloressigsäureester. Die Umsetzung kann, insbesondere bei Verwendung von Chloressigsäurehalogeniden, in An- oder Abwesenheit von säurebindenden Mitteln durchgeführt werden. Als solche kommen in Betracht:
tertiäre Amine, wie z.B. Triäthylamin und Pyridin, anorganische Basen wie Oxide, Hydroxide, Carbonate bzw. Hydrogencarbonate. Als säurebindendes Mittel kann ferner im Überschuß angesetztes Anilin der Formel II dienen.

Die Herstellung von Verbindungen der Formel II erfolgt durch Umsetzung der bereits erwähnten 5-Halogenalkyl-1,2,4-triazole der Formel III mit Anilinen der Formel IV in Gegenwart von säurebindenden Mitteln:

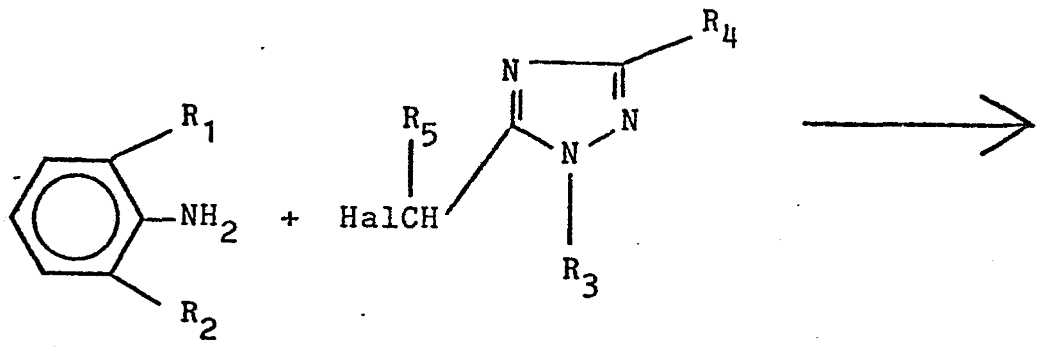

IV III

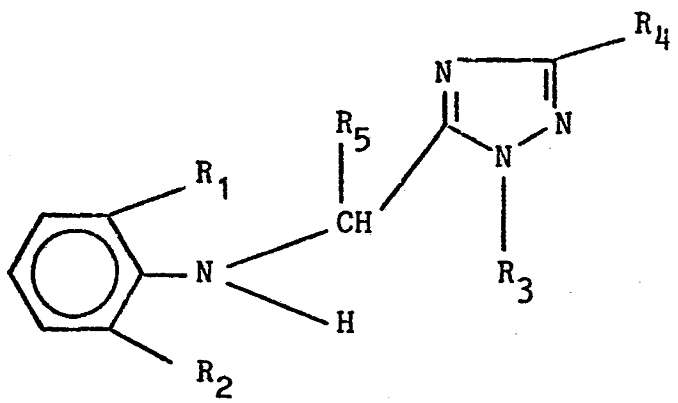

II

Hal bedeutet Halogen, bevorzugt Chlor und Brom.

Die Umsetzung wird in gegenüber den Reaktionsteilnehmern inerten Lösungs- oder Verdünnungsmitteln oder aber in überschüssigem Anilin der Formel IV durchgeführt. Als Lösungsmittel kommen in Frage: Aliphatische, aromatische oder halogenierte Kohlenwasserstoffe wie Benzol, Toluol, Xylole, Petroläther, Chlorbenzol, Methylenchlorid, Äthylenchlorid, Chloroform; Äther wie Dialkyläther, Dioxan, Tetrahydrofuran; Nitrile wie Acetonitril, N,N-dialkylierte Amide wie Dimethylformamid; ferner Dimethylsulfoxid sowie Gemische dieser Lösungsmittel untereinander. Die Umsetzungstemperaturen liegen zwischen 60°C und dem Siedepunkt des verwendeten Lösungsmitteln, vorzugsweise zwischen 100 und 160°C.

BAD ORIGINAL

Als säurebindende Mittel kommen tertiäre Amine, wie z.B. Triäthylamin, Pyridin oder anorganische Basen wie Oxide oder Hydroxide und Carbonate bzw. Hydrogencarbonate von Alkali- und Erdalkalimetallen in Frage. Als säurebindendes Mittel kann aber auch im Überschuß eingesetztes Anilin der Formel IV dienen.

Die zur Herstellung der Verbindungen II benötigten Aniline IV sind allgemein bekannte Verbindungen der organischen Chemie. Als Beispiele seien genannt: 2-Methylanilin, 2-Isopropylanilin, 2,6-Dimethylanilin, 2-Methyl-6-äthylanilin, 2,6-Diäthylanilin, 2-Äthyl-6-isopropylanilin, 2-Methyl-6-isopropylanilin, 2,6-Diisopropylanilin.

Aufgrund der gehinderten Rotation um die N-C(Phenyl)-Achse treten bei $R_1 \neq R_2$ Enantiomere auf. Für $R_5 \neq H$ treten im Falle von $R_1 = R_2$ Enantiomere, im Falle von $R_1 \neq R_2$ zwei Diastereomerenpaare auf. Die Erfindung betrifft sowohl die freien Enantiomere und Diastereomere(npaare) als auch deren Gemische.

Die Verbindungen der allgemeinen Formel I besitzen eine hervorragende herbizide Wirkung gegen annuelle und perennierende Schadpflanzen. Unter den bekämpfbaren Arten befinden sich wirtschaftlich wichtige Schadgräser wie Setaria (Borstenhirse), Digitaria (Fingerhirse), Echinochloa (Hühnerhirse); Cyperaceen (Sauergräser) und Agropyron (Quecke) sowie auch dikotyle Unkräuter wie Matricaria (Kamille), Chrysanthemum (Saatwucherblume) und Amaranthus (Amaranth). Daneben schonen die Verbindungen in den herbizid wirksamen Aufwandmengen viele Kulturpflanzenarten, so daß sie in wichtigen Großkulturen zur Bekämpfung von grasartigen und breitblättrigen Unkräutern eingesetzt werden können.

So kann man mit den erfindungsgemäßen Mitteln beispielsweise

BAD ORIGINAL

Hühnerhirse und Amaranthus in Mais und Borstenhirse und Fingerhirse in Soja- und Baumwollkulturen wirksam bekämpfen, ohne daß ein Schaden an den Kulturpflanzen auftritt. Darüberhinaus können sie auch in breitblättrigen Kulturen der gemäßigten Breiten, z.B. in Raps, selektiv eingesetzt werden, beispielsweise zur Bekämpfung vonAusfallgetreide. Hinsichtlich der Selektivität sind die neuen Verbindungen bekannten Pflanzenschutzmitteln ähnlicher Struktur wie 2,6-Dimethyl-N-methoxymethyl-chloracetanilid (Alachlor) und 2-Methyl-6-ethyl-N-methoxymethyl-chloracetanilid (Metolachlor) bei einer Reihe von Pflanzen überlegen.

Die erfindungsgemäßen Verbindungen zeigten ferner sowohl bei Vorauflauf- wie bei Nachauflaufanwendungen gute Wirksamkeit beim Einsatz als Wachstumsregulatoren. So kann z.B. das Wachstum von Kuturgräsern (Reis, Getreide usw.) und von dikotylen Kulturpflanzen günstig beeinflußt werden. Auf diese Weise lassen sich erhebliche Ertragssteigerungen an landwirtschaftlichen und gärtnerischen Kulturen erzielen. Schließlich können die Verbindungen auch als Keimhemmungsmittel verwendet werden.

Die erfindungsgemäßen Mittel enthalten die Wirkstoffe der Formel I im allgemeinen zu 2 bis 95 Gew.-%. Sie können als Spritzpulver, emulgierbare Konzentrate, versprühbare Lösungen, Stäubemittel oder Granulate in den üblichen Zubereitungen angewendet werden.

Spritzpulver sind in Wasser gleichmäßig dispergierbare Präparate, die neben dem Wirkstoff außer einem Verdünnungsmittel oder Inertstoff noch Netzmittel, z.B. polyoxethylierte Alkylphenyl, polyoxethylierte Fettalkohole, Alkyl- oder Alkylphenyl-sulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium, enthalten.

BAD ORIGINAL

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Amulgatoren können beispielsweise verwendet werden:

Alkylarylsulfonsaure Kalziumsalze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxethylensorbitan-fettsäureester oder Polyoxethylen-sorbitester.

Stäubemittel erhält man durch Vermahlen des Wirkstoffes mit fein verteilten, festen Stoffen, z.B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mieralölen auf die Oberfläche von Trägerstoffen wie Sand Kaolinite oder von granuliertem Inertmaterial. Auch können geeignete Wirkstoffe in der für die Herstellung von Düngemittelgranalien üblichen Weise - gewünschtenfalls in Mischung mit Düngemitteln - hergestellt werden.

Bei herbiziden Mitteln können die Konzentrationen der Wirkstoffe in den handelsüblichen Formulierungen verschieden sein.

In Spritzpulvern variiert die Wirkstoffkonzentration z.B. zwischen etwa 10 % und 80 %, der Rest besteht aus den oben angegebenen Formulierungszusätzen. Bei emulgierbaren Konzentraten kann die Wirkstoffkonzentration gleichfalls etwa 10 %

BAD ORIGINAL

bis 80 % betragen. Stäubeformige Formulierungen enthalten meistens 5 bis 20 % an Wirkstoff, versprühbare Lösungen etwa 2 bis 20 %. Bei Granulaten hängt der Wirkstoffgehalt zum Teil daon ab, ob die wirksame Verbindung flüssig oder fest vorliegt und welche Granulierhielsmittel, Füllstoffe usw. verwendet werden.

Zur Anwendung werden die handelsüblichen Konzentrate gegebenenfalls in üblicher Weise verdünnt, z.B. bei Spritzpulvern und emulgierbaren Konzentraten mittels Wasser. Stäubeförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit u.a., variiert die erforderliche Aufwandmenge. Sie beträgt im allgemeinen zwischen 0,1 und 10 kg/ha, vorzugsweise etwa 0,1 bis 5,0 kg/ha Wirkstoff. Der erfindungsgemäße Wirkstoff kann mit anderen Herbiziden, Insektiziden und Fungiziden kombiniert werden.

## HERSTELLUNGSBEISPIELE

### Beispiel 1

N-(1,3-Dimethyl-1,2,4-triazol-5-ylmethyl)-chloressigsäure-2,6-dimethylanilid

16 g (0.07 Mol) N-(1,3-Dimethyl-1,2,4-triazol-5-yl-methyl)-2,6-dimethylanilin (Beispiel 1a) wurden in 100 ml Toluol gelöst und die Reaktionsmischung auf 100°C erhitzt. Bei dieser Temperatur gibt man 8.4 g (0.074 Mol) Chloracetylchlorid innerhalb von 15 Minuten zu und rührt 2 Stunden bei 110°C nach. Nach Abkühlung wird das entstandende Hydrochlorid durch Zugabe von 5 %iger Natronlauge freigesetzt, die organische Phase abgetrennt und Toluol abdestilliert. Der verbleibende Rückstand wird aus Toluol umkristallisiert. Man erhält 18.5 g (86.5 %) N-(1.3-Dimethyl-1,2,4-triazol-5-

BAD ORIGINAL

ylmethyl)-chloressigsäure-2,6-dimethylanilid mit dem Schmelzpunkt 106 bis 108°C.

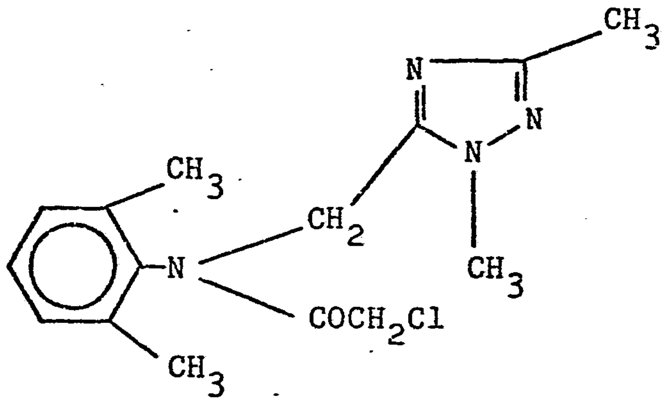

In analoger Weise wurden die in Tabelle 1 aufgeführten Wirkstoffe der Formel I hergestellt.

Tabelle 1

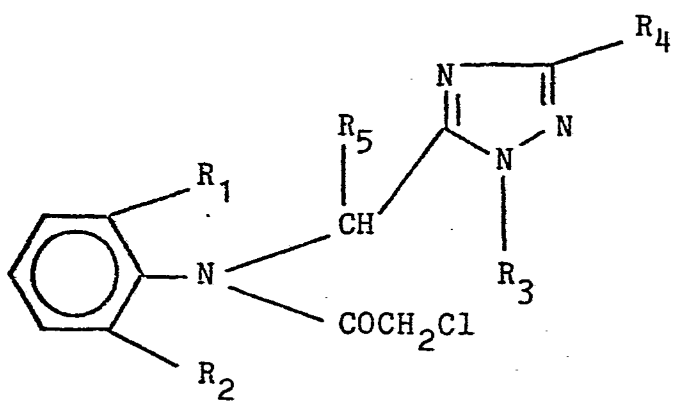

I

| Beisp. Nr. | $R_1$ | $R_2$ | $R_3$ | $R_4$ | $R_5$ | physikal. Daten |
|---|---|---|---|---|---|---|
| 2 | $CH_3$ | $CH_3$ | $CH_3$ | $C_2H_5$ | H | Fp. 63°C |
| 3 | $CH_3$ | $C_2H_5$ | $CH_3$ | $C_2H_5$ | H | |
| 4 | $CH_3$ | $CH_3$ | $C_2H_5$ | $C_2H_5$ | H | Fp. 102-103.5°C |
| 5 | $C_2H_5$ | $C_2H_5$ | $CH_3$ | $CH_3$ | H | |
| 6 | $CH_3$ | $CH_3$ | $i-C_3H_7$ | $C_2H_5$ | H | Fp. 88-88.5°C |

BAD ORIGINAL

Tabelle 1 (Fortsetzung)

| Beisp. Nr. | $R_1$ | $R_2$ | $R_3$ | $R_4$ | $R_5$ | physikal. Daten |
|---|---|---|---|---|---|---|
| 7 | $CH_3$ | $C_2H_5$ | $CH_3$ | $C_2H_5$ | H | $n_D^{23}$ = 1.5441 |
| 8 | $C_2H_5$ | $C_2H_5$ | $CH_3$ | $C_2H_5$ | H | $n_D^{23}$ = 1.5417 |
| 9 | $CH_3$ | $CH_3$ | $CH_3$ | n-$C_3H_7$ | H | |
| 10 | $CH_3$ | $C_2H_5$ | i-$C_3H_7$ | $CH_3$ | H | |
| 11 | $CH_3$ | $C_2H_5$ | $CH_3$ | $CH_3$ | $CH_3$ | Fp. 144°C (Diastereomeren-paargemisch) bzw. 164°C (reines Diastereomerenpaar) |
| 12 | $CH_3$ | $CH_3$ | i-$C_3H_7$ | H | H | Fp. 113°C |
| 13 | $CH_3$ | $CH_3$ | $CH_3$ | H | H | Fp. 113°C |

Beispiel 1a (Ausgangsstoffe)

N-(1,3-Dimethyl-1.2.4-triazol-5-ylmethyl)-2,6-dimethylanilin

96.8 g (0.8 Mol) 2,6-Dimethylanilin und 28 g (0.193 Mol) 1.3-Dimethyl-5-chlormethyl-1.2.4-triazol werden 30 Stunden bei 95°C gerührt. Anschließend wird mit 200 ml Toluol verdünnt, auf 5°C gekühlt und mit 5 %iger Natronlauge unter Eiskühlung alikalisch gestellt. Nach Abtrennung der organischen Phase wird zunächst Toluol abdestilliert und der verbleibende Rückstand im Vakuum fraktioniert destilliert. Nach Abnahme von 2,6-Dimethylanilin als Vorlauf bei 45°C, 0,013 mbar erhält man 31.95 g (72 % d.Th.) N-(1,3-Dimethyl-1.2.4-triazol-5-ylmethyl)-2,6-dimethylanilin mit dem Siedepunkt 135°C, 0.013 mbar (gaschromat. Reinheit 96.7 %).

BAD ORIGINAL

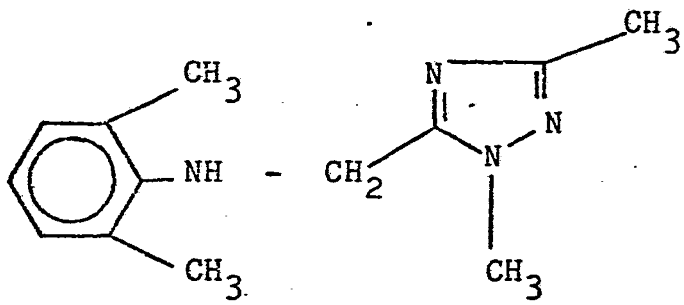

In analoger Weise wurden die in Tabelle 2 aufgeführten Verbindungen erhalten.

Tabelle 2

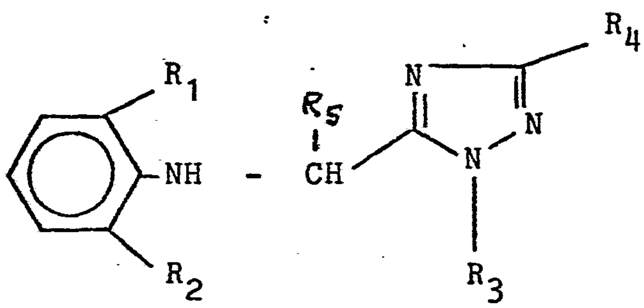

II

| Beisp. Nr. | $R_1$ | $R_2$ | $R_3$ | $R_4$ | $R_5$ | physikal. Daten |
|---|---|---|---|---|---|---|
| 2a | $CH_3$ | $CH_3$ | $CH_3$ | $C_2H_5$ | H | $Kp_{0.013\ mb}$ 141°C |
| 3a | $CH_3$ | $C_2H_5$ | $CH_3$ | $CH_3$ | H | |
| 4a | $CH_3$ | $CH_3$ | $C_2H_5$ | $CH_5$ | H | Fp. 74-75°C |
| 5a | $C_2H_5$ | $C_2H_5$ | $CH_3$ | $CH_3$ | H | |
| 6a | $CH_3$ | $CH_3$ | i-$C_3H_7$ | $CH_3$ | H | Fp. 88-89 °C |
| 7a | $CH_3$ | $C_2H_5$ | $CH_3$ | $C_2H_5$ | H | $Kp_{0.065\ mb}$ 137°C |
| 8a | $C_2H_5$ | $C_2H_5$ | $CH_3$ | $C_2H_5$ | H | $n_D^{25}$= 1.5352 |
| 9a | $CH_3$ | $CH_3$ | $CH_3$ | n-$C_3H_7$ | H | |
| 10a | $CH_3$ | $C_2H_5$ | i-$C_3H_7$ | $CH_3$ | H | |
| 11a | $CH_3$ | $C_2H_5$ | $CH_3$ | $CH_3$ | $CH_3$ | Fp. 79°C |
| 12a | $CH_3$ | $CH_3$ | i-$C_3H_7$ | H | H | Kp. 216°C |
| 13a | $CH_3$ | $CH_3$ | $CH_3$ | H | H | Fp. 132°C (als Hydrochlorid) |

BAD ORIGINAL

## FORMULIERUNGSBEISPIELE

### Beispiel A:

Ein emulgierbares Konzentrat wird erhalten aus

15 Gew.-Teilen Wirkstoff
75 Gew.-Teilen Cyclohexan als Lösungsmittel
und 10 Gew.-Teilen oxäthyliertes Nonylphenol (10 AeO) als Emulgator.

### Beispiel B:

Ein in Wasser leicht dispergierbares benetzbares Pulver wird erhalten, indem man

25 Gew.-Teile Wirkstoff
64 Gew.-Teile kaolinhaltiges Quarz als Inertstoff
10 Gew.-Teile ligninsulfonsaures Kalium
und 1 Gew.-Teile oleylmethyltaurinsaures Natrium als Netz- und Dispergiermittel

mischt und in einer Stiftmühle mahlt.

### Beispiel C:

Ein Stäubemittel wird erhalten, indem man

10 Gew.-Teile Wirkstoff
und 90 Gew.-Teile Talkum als Inertstoff

mischt und in einer Schlagmühle zerkleinert.

### Beispiel D:

Ein Granulat besteht z.B. aus etwa

BAD ORIGINAL

2 bis 15 Gew.-Teilen Wirkstoff
98 bis 85 Gew.-Teilen inerten Granulatmaterialien, wie z.B. Attapulgit, Bimsstein und Quarzsand.

## BIOLOGISCHE BEISPIELE

### Beispiel 1

Samen verschiedener Unkräuter und Ungräser wurden in Plastiktöpfen in sandigem Lehmboden ausgesät und mit einer Schicht Sandboden abgedeckt. Die als Spritzpulver formulierten erfindungsgemäßen Verbindungen wurden in Form von wäßrigen Suspensionen bzw. emulsionen auf die Bodenoberfläche gesprüht. Die Wasseraufwandmenge betrug dabei umgerechnet 600 l/ha. Anschließend wurden die Töpfe im Gewächshaus unter günstigen Wuchsbedingungen aufgestellt. Nach 4 Wochen wurde die prozentuale Abtötung bzw. Schädigung der Versuchspflanzen im Vergleich zu unbehandelten Kontrollen visuell bonitiert.

### Tabelle 1

Herbizide Vorauflaufwirkung der erfindungsgemäßen Verbindungen. Wirkung in % im Vergleich zu unbehandelten Kontrollen.

| Verb. Bsp. | Dosis (kg/ha) | herbizide Wirkung | | | | |
|---|---|---|---|---|---|---|
| | | AVF | ALM | SAL | LOM | MAI |
| | 2,4 | 99 | 99 | 100 | 100 | 100 |
| 1 | 0,6 | 95 | 98 | 100 | 100 | 100 |
| | 0,15 | | | | | 85 |
| | 2,4 | 100 | 100 | 100 | 100 | 100 |
| 2 | 0,6 | 97 | 100 | 100 | 100 | 99 |
| | 0,15 | | | | | 70 |

BAD ORIGINAL

Tabelle 1 (Fortsetzung)

| Verb. Bsp. | Dosis (kg/ha) | herbizide Wirkung AVF | ALM | SAL | LOM | MAI |
|---|---|---|---|---|---|---|
| | 2,4 | 100 | 100 | 100 | 100 | 100 |
| 3 | 0,6 | 99 | 100 | 100 | 100 | 98 |
| | 0,15 | | | | | 100 |
| | 2,4 | 100 | 100 | 100 | 100 | 100 |
| 8 | 0,6 | 94 | 98 | 100 | 99 | 100 |
| | 0,15 | | | | | |
| | 2,4 | 85 | 85 | 100 | 99 | - |
| 6 | 0,6 | - | - | 95 | 80 | - |
| | 0,15 | | | | | |
| 12 | 2.4 | - | - | - | 100 | - |
| 13 | 2.4 | - | - | - | 100 | - |

Abkürzungen zu Tabelle 1:

AVF = Avena fatua
ALM = Alopecurus myosuroides
SAL = Setaria lutescens
LOM = Lolium multiflorum
MAI = Matricaria inodora

Die Ergebnisse belegen die guten herbiziden eigenschaften der erfindungsgemäßen Verbindunegn der Formel (I) gegen monokotyle und dikotyle Schadpflanzen und ihre Überlegenheit. Im Vergleich zu dem bisherigen Stand der Technik (2,6-Diethyl-N-methoxymethyl-chloracetanilid) weisen die erfindungsgemäßen wirkstoffe eine erheblich bessere herbizide Wikrung insbesondere gegen wirtschaftlich wichtige Ungräser wie Avena fatua, Alopecurus myosuroides, Setaria

BAD ORIGINAL

lutescens und Lolium multiflorum auf.

Beispiel 2:

Nach der gleichen Methode wie in Beispiel 1 beschrieben wurden Samen bzw. Rhizomstücke verschiedener Unkräuter und Kulturpflanzen in Töpfen ausgelegt und im Vorauflaufverfahren mit den erfindungsgemäßen Verbindungen der Formel I behandelt. Nach 4 Wochen Standzeit im Gewächshaus wurden die Pflanzen hinsichtlich Schädigung wie in Beispiel 1 bonitiert.

Tabelle 2 gibt eine Zusammenfassung der erzielten herbiziden Wirkungen im Vorauflauf. Die angegebenen Werte zeigen klar, daß die erfindungsgemäßen Verbindungen neben sehr guter herbizider Wirksamkeit gegen mon- und dikptyle Problemunkräuter eine gute Verträglichkeit bei vielen Kulturpflanzen aufweisen und deshalb zur selektiven Unkrautbekämpfung in landwirtschaftlich bedeutenden Kulturen geeignet sind.

BAD ORIGINAL

Tabelle 2

Herbizide Wirkung und Selektivität der erfindungsgemäßen Verbindungen bei Vorauflaufapplikation. Wirkung in %.

| Verbindung Bsp.-Nr. | Dosis (kg/ha) | herbizide Wirkung | | | | | | | |
|---|---|---|---|---|---|---|---|---|---|
| | | SAF | SRH | CYE | BA | BN | GS | MS | GH |
| 1 | 2,4 | 100 | 100 | 100 | 50 | 0 | 80 | 20 | 30 |
| | 0,6 | 100 | 100 | 100 | 30 | 0 | 30 | 10 | 20 |
| 2 | 2,4 | 100 | 99 | 100 | 60 | 10 | 85 | 98 | 20 |
| | 0,6 | 100 | 95 | 95 | 30 | 5 | 5 | 60 | 10 |
| Metolachlor | 2,4 | 100 | 100 | 100 | 100 | 100 | 100 | 100 | 50 |
| | 0,6 | 98 | 90 | 70 | 100 | 100 | 98 | 100 | 10 |

Abkürzungen zu Tabelle 2:

SAF = Setaria faberi
SRH = Sorghum halepense
CYE = Cyperus esculentus
BA = Beta altissima
BN = Brassica napus
GS = Glycine soja
MS = Medicago sativa
GH = Gossypium hirsutum

BAD ORIGINAL

Patentansprüche:

1. Chloracetanilide der allgemeinen Formel I

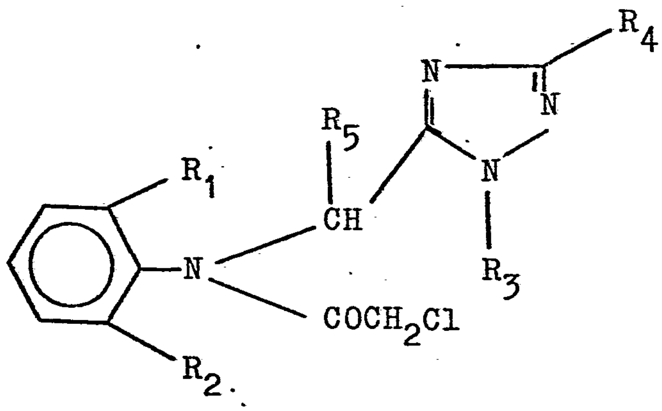

I

worin
$R_1$ Methyl, Äthyl oder Isopropyl,
$R_2$ Wasserstoff, Methyl, Äthyl oder Isopropyl,
$R_3$ $(C_1-C_3)$-Alkyl,
$R_4$ Wasserstoff oder $(C_1-C_3)$-Alkyl, und
$R_5$ Wasserstoff oder Methyl bedeuten.

2. Verfahren zur Herstellung von Verbindungen der Formel I, dadurch gekennzeichnet, daß man N-substituierte Aniline der Formel II

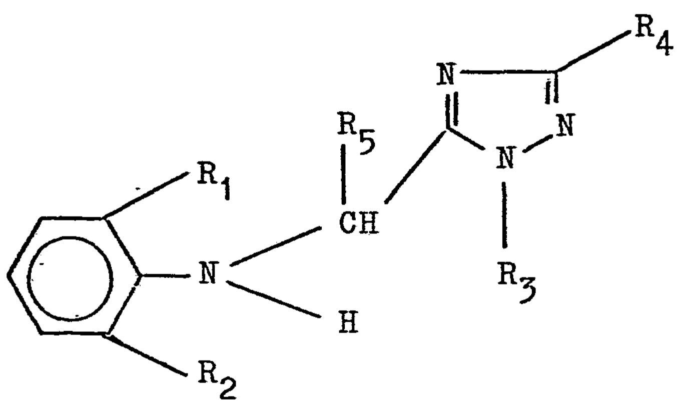

II

mit Chloracetylierungsmitteln behandelt.

3. Herbizide und wachstumsregulierende Mittel, gekennzeichnet durch einen Gehalt an Verbindungen der Formel I.

BAD ORIGINAL
BAD ORIGINAL

4. Verwendung von Verbindungen der Formel I zur Bekämpfung von unerwünschtem Pflanzenwuchs.

5. Verwendung von Verbindungen der Formel I zur Wachstumsregulierung von Kulturpflanzen.

6. Verfahren zur Bekämpfung von unerwünschtem Pflanzenwuchs und zur Wachstumsregulierung, dadurch gekennzeichnet, daß man auf die zu behandelnde Fläche eine wirksame Menge einer Verbindung der Formel I aufbringt.

BAD ORIGINAL

Europäisches Patentamt

## EUROPÄISCHER RECHERCHENBERICHT

EP 82 10 4460

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | EP-A-0 003 539 (BAYER) *Seiten 56-58* | 1-6 | C 07 D 249/08<br>A 01 N 43/64 |
| | --- | | |
| X | GB-A-2 020 657 (CHEVRON) *Seiten 1 bis 3, 7 bis 10* | 1-6 | |
| | --- | | |
| X | EP-A-0 010 163 (BAYER) *Seiten 1 bis 5* & DE - A 2 842 284 (Cat. D,X) | 1-6 | |
| | ----- | | |
| | | | **RECHERCHIERTE SACHGEBIETE (Int. Cl. 3)** |
| | | | C 07 D 249/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 30-10-1982 | DE BUYSER I.A.F. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03.82